# EUROPEAN PATENT APPLICATION

(11) **EP 3 427 757 A2**
(43) Date of publication of application: **16.01.2019**
(21) Application number: 18182211.5
(22) Date of filing: 06.07.2018
(51) Int. Cl.: A61L 2/04, A61L 2/18, B65B 55/10, B65B 55/06

(54) **A STERILIZATION APPARATUS FOR STERILIZING A WEB OF PACKAGING MATERIAL AND A STERILIZATION METHOD**

(30) Priority: 10.07.2017 EP 17180496
(71) Applicant: Tetra Laval Holdings & Finance S.A., 1009 Pully (CH)
(72) Inventor: Saeidihaghi, Arash, 247 35 SÖDRA SANDBY (SE); Löfvall Gustavsson, Peter, SE29170 Kristianstad (SE)
(74) Representative: Tetra Pak - Patent Attorneys SE

(57) **Abstract**

A sterilization apparatus for sterilizing a web of packaging material being conveyed along a path thereof, in an upstream- to a downstream direction, is disclosed. The sterilization apparatus comprises a sterilizing module in which a sterilizing agent is applied to the web, a sterilization chamber through which the web is fed when conveyed downstream of the sterilizing module, a heating unit configured to heat the web in a heating medium when fed through the sterilization chamber, and a pressurized gas outlet arranged upstream of the heating unit and downstream of the sterilizing module, wherein the pressurized gas is configured to expel a pressurized gas, having a lower temperature than said heating medium, towards the web. A method of sterilizing a web of packaging material and a related packaging machine are also disclosed.

## Description

### Technical Field

The present invention relates to a sterilization apparatus for sterilizing a web of packaging material, a method of sterilizing a web of packaging material, and a related packaging machine.

### Background

Within packaging technology, packages for packing and transporting various pourable products are produced from a laminated packaging material comprising a core layer of, for example, paper or paperboard and an outer, liquid-tight coating of thermoplastic material on at least that side of the core layer which forms the inside of the package. Sometimes the material also includes a gas barrier, for example in the form of an aluminum layer. Such packaging containers are often produced from a web of packaging material that is formed into a tube by overlappingly sealing the longitudinal edges of the web. The tube is continuously filled with a product and then transversally sealed and formed into a chain of individual packages. To extend the shelf-life of the products being packed the web is sterilized before the forming and filling operations. Depending on how long shelf-life is desired and whether the distribution and storage is made in chilled or ambient temperature, different levels of sterilization can be chosen. One way of sterilizing a web is chemical sterilization with a sterilization agent using for example a bath of hydrogen peroxide. After passing the sterilization bath, the packaging material web is normally subjected to heat in order to sterilize the web additionally and/or to remove excess sterilizing agent.

It is desirable to improve previous sterilizing apparatuses and methods, to achieve a further optimized sterilization process of packaging containers. Also, in case the packaging material comprises opening elements provided by e.g. injection molding into the packaging material, is desirable to facilitate the removal of sterilizing residues that may be deposited in such opening elements. It is also desirable to facilitate complying with regulations concerning residues of sterilizing agent on the packaging material, and also to comply with microbiological requirements.

Hence, an improved sterilization apparatus for sterilizing a web of packaging material would be advantageous and in particular allowing for avoiding more of the above-mentioned problems and compromises. An improved method of sterilizing a web of packaging material would be advantageous.

### Summary

Accordingly, examples of the present invention preferably seek to mitigate, alleviate or eliminate one or more deficiencies, disadvantages or issues in the art, such as the above-identified, singly or in any combination by providing a device according to the appended patent claims.

According to a first aspect a sterilization apparatus for sterilizing a web of packaging material being conveyed along a path thereof, in an upstream- to a downstream direction, is provided. The sterilization apparatus comprises a sterilizing module in which a sterilizing agent is applied to the web, a sterilization chamber through which the web is fed when conveyed downstream of the sterilizing module, a heating unit configured to heat the web in a heating medium when fed through the sterilization chamber, and a pressurized gas outlet arranged upstream of the heating unit and downstream of the sterilizing module, wherein the pressurized gas outlet is configured to expel a pressurized gas, having a lower temperature than said heating medium, towards the web.

According to a second aspect a method of sterilizing a web of packaging material is provided. The method comprises applying a sterilizing agent to the web, expelling a pressurized gas towards the web subsequent of applying the sterilizing agent, and heating the web in a sterilizing chamber with a heating medium subsequent of expelling the pressurized gas towards the web, wherein the pressurized gas has a lower temperature than said heating medium.

According to a third aspect a packaging machine is provided for producing sealed packages of pourable food products from a web of packaging material. The packaging machine carries out a method of sterilizing a web of packaging material according to the second aspect, and/or comprises a sterilization apparatus according to the first aspect.

According to a fourth aspect a computer program product is provided comprising instructions which, when the program is executed by a computer, cause the computer to carry out the steps of the method according the second aspect.

Further examples of the invention are defined in the dependent claims, wherein features for the second, third, and fourth aspects of the disclosure are as for the first aspect mutatis mutandis.

Some examples of the disclosure provide for improved sterilization of a packaging material.

Some examples of the disclosure provide for improved sterilization of a packaging material comprising opening elements provided by e.g. injection molding into the packaging material.

Some examples of the disclosure provide for improved removal of residues of sterilizing agent from the packaging material.

Some examples of the disclosure provide for avoiding insufficient sterilization due to excess heating of the packaging material.

Some examples of the disclosure provide for a more controlled evaporation of a sterilizing agent from the packaging material.

Some examples of the disclosure provide for improved control of the temperature in the sterilization method of a packaging material.

Some examples of the disclosure provide for complying with regulations of residues of sterilizing agent on the packaging material.

Some examples of the disclosure provide for complying with microbiological requirements.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which examples of the invention are capable of will be apparent and elucidated from the following description of examples of the present invention, reference being made to the accompanying drawings, in which;
Fig. 1 is a schematic illustration of a sterilization apparatus according to examples of the disclosure;
Fig. 2 is a schematic illustration of a sterilization apparatus according to examples of the disclosure;
Fig. 3 is a schematic illustration of a sterilization apparatus according to examples of the disclosure;
Fig. 4a is a flowchart of a method of sterilizing a web of packaging material according to examples of the disclosure; and
Fig. 4b is a flowchart of a method of sterilizing a web of packaging material according to examples of the disclosure.

### Detailed Description

Specific examples of the invention will now be described with reference to the accompanying drawings. This invention may, however, be embodied in many different forms and should not be construed as limited to the examples set forth herein; rather, these examples are provided so that this disclosure will be thorough and complete, and will fully convey the scope of the invention to those skilled in the art. The terminology used in the detailed description of the examples illustrated in the accompanying drawings is not intended to be limiting of the invention. In the drawings, like numbers refer to like elements.

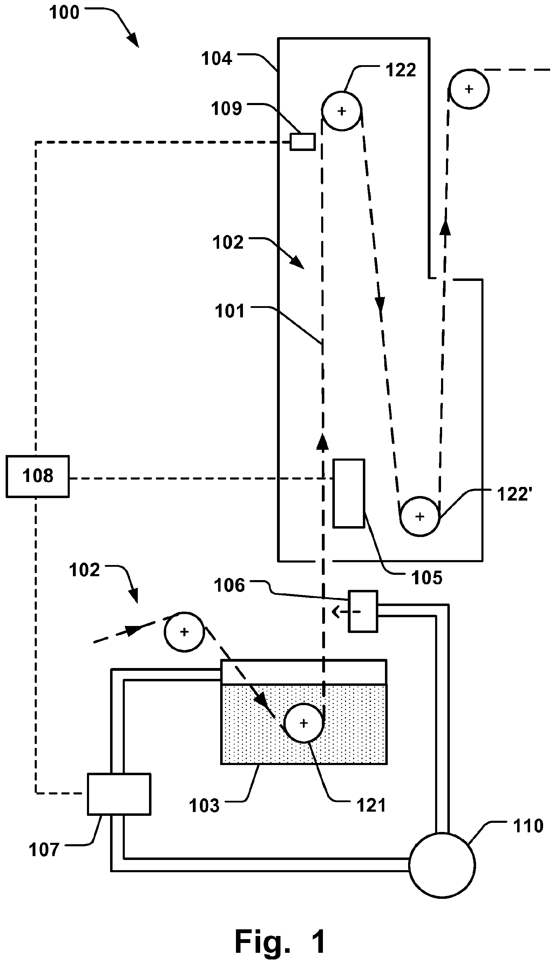

Fig. 1 is a schematic illustration of a sterilization apparatus 100 for sterilizing a web 101 of packaging material being conveyed along a path 102 thereof in an upstream- to a downstream direction, i.e. from an entry location into the sterilization apparatus 100 to an exit location. The sterilization apparatus 100 comprises a sterilizing module 103 in which a sterilizing agent is applied to the web, and a sterilization chamber 104 through which the web 101 is fed when conveyed downstream of the sterilizing module 103, i.e. after the sterilizing agent has been applied to the web in the sterilizing module 103. The sterilization apparatus 100 comprises a heating unit 105 configured to heat the web 101 in a heating medium when fed through the sterilization chamber 104. Fig.1 illustrates one heating unit 105 but other heating units may also be provided in the sterilization chamber along the path 102. After passing the sterilization module 103 the web 101 is covered by sterilizing agent, and heating of the web 101 provides for increasing the potency of the sterilizing agent and also for evaporation thereof when passing through the sterilization apparatus 100. The sterilization apparatus 100 further comprises a pressurized gas outlet 106 arranged upstream of the heating unit 105 and downstream of the sterilizing module 104, i.e. between the heating unit 105 and the sterilizing module 104 in the path 102 along which the web 101 is conveyed. Upstream of the heating unit 105 should be construed as arranged before the heating unit 105 and/or before the environment in which the heating unit 105 provides with heating medium for heating of the web 101 (although the heating unit may comprise elements physically arranged before the pressurized gas outlet 106 along the path 102). The pressurized gas outlet 106 is configured to expel a pressurized gas, having a lower temperature than the mentioned heating medium, towards the web 101. Having a pressurized gas outlet 106 arranged after the web 101 passing the sterilizing module 103 and before being subjected to heating in the heating medium in the sterilization chamber 104 provides for temperature control of the web 101 in an intermediate process while maintaining optimized conditions in the sterilizing module 103 for the application of the sterilizing agent, such as an optimized temperature therein, and for optimized conditions in subsequent steps such as exposing the web 101 to a certain pressure (e.g. a fan blowing against the web 101) for removal of sterilizing agent residues. In this case, while it may be necessary to expose the web 101 to a certain pressure for removal of sterilizing agent residues, having a pressurized gas outlet 106 arranged to expel a pressurized gas, having a lower temperature than the mentioned heating medium, to achieve an intermediate temperature control of the web 101 provides for allowing an optimization of both the pressure and temperature for such residue removal (e.g. pools of wet sterilizing agent) without the risk of overheating the web 101 when exposed to the heating medium when fed through the sterilization chamber 104. Such excess heating can otherwise cause too fast evaporation of the sterilizing agent, i.e. a shortened time during which the sterilizing agent is in contact with the web 101 of packaging material. By expelling a pressurized gas having a lower temperature than the heating medium towards the web 101 such that the temperature thereof can be controlled at the onset of the treatment thereof in the sterilization chamber 104 thus provides for minimizing the risk of too fast removal of sterilizing agent, while at the same time optimized conditions can be maintained for removal of e.g. pools or droplets of sterilizing agent in cavities or recesses of injection molded opening mechanisms on the web 101 after passing the sterilizing module 103. By expelling the mentioned pressurized gas towards the web 101 after passing the sterilizing module 103 is it possible to achieve a smooth temperature ramp along the path 102 in the sterilization chamber 104 which provides for demonstrating good microbiological test results of the web 101. The temperature of the web 101 may be ramped to about 75 degrees C. The pressurized gas outlet 106 may expel a pressurized gas at bout 30-40 degrees C, to achieve a smooth temperature ramp and a particularly advantageous sterilization process. The pressurized gas outlet 106 may be arranged outside the environment of the sterilization chamber 104 heated by the heating medium. A more efficient temperature control of the web 101 may thus be proved without interference from the environment in the sterilization chamber 104. It is conceivable however that the pressurized gas outlet 106 and temperature control of the web 101 may be incorporated into the heated environment of the sterilization chamber 104 while still providing for the advantageous effects as described above.

Having the pressurized gas outlet 106 arranged as described above thus provides for increasing the control of the evaporation process of a sterilizing agent from the packaging material. In this manner, complying with microbiological requirements is facilitated, due to the improved sterilization process, in particular for more complex opening mechanisms, such as injection molded mechanisms, pre-formed in the web of packaging material 101. At the same time, the control of the amounts of sterilizing agent residues in such opening mechanisms is improved, thus facilitating the process of complying with regulations of residues of sterilizing agent on the packaging material.

The sterilizing module 103 may comprise a bath of a sterilizing agent, such as hydrogen peroxide, wetting the web 101 passing therethrough.

As schematically illustrated in Fig. 1, the sterilization apparatus 100 may comprise a temperature regulator 107 being in communication with the pressurized gas outlet 106, and a control unit 108 connected to the temperature regulator 107 and to at least one temperature sensor 109 in the sterilization chamber 104, adjacent the path 102 in which the web 101 is conveyed, to receive temperature data therefrom. The control unit 108 may be configured to control the temperature of the pressurized gas based on the temperature data so that the temperature of the web 101 of packaging material assume predefined values along the path 102 in which the web 101 is conveyed. The evaporation of the sterilizing agent can thus be effectively controlled, e.g. ensuring that the sterilizing agent is in contact with the web 101 for a desired amount of time, and at the same time minimize the amount of sterilizing agent residues at the end of the path 102 in the sterilization chamber 104. Both the temperature and the pressure of the pressurized gas may be adjusted based on the received temperature data. Placing a temperature sensor 109 adjacent, or inside, a conveyor element 122 (such as a roller) arranged at an opposite end of the sterilization chamber 104, relative an inlet thereof may provide for particularly advantageous temperature data for controlling the pressurized gas as well as the heating unit 105. A series of temperature sensors may however be provided in the sterilization chamber 104 along the path 102 to accurately characterize the temperature profile therein. Conveyor element 122 may be arranged opposite a first conveyor element 121 arranged in the sterilizing module 103, and opposite a second conveyor 122' arranged at an opposite end of the sterilization chamber 104 as schematically illustrated in Fig. 1. The conveyor elements 121, 122, 122', may comprise rollers for transporting the web 101 of packaging material. The temperature regulator 107 may comprise a heat exchanger or any other regulator configures to provide different temperatures of the cooling medium.

The sterilization apparatus 100 may comprise a pump 110 in communication with the pressurized gas outlet 106 and being configured to pressurize a gas for propagation towards the web 102. The control unit 108 may be configured to control the pressure of the pressurized gas being expelled from the pressurized gas outlet based on the temperature data. Thus, the pressure of the gas may be optimized to provide for the required temperature control of the web 101 so that the evaporation of the sterilizing agent along the path 102 can be controlled, while at the same time removing residues of sterilizing agent such as pools or droplets thereof being deposited at e.g. injection molded opening elements of the web 101. The pressurized gas outlet 106 may comprise a fan that blows the pressurized gas such as air against the web 101, to remove such residues. Fig. 1 illustrates an example where the pump 110 is located downstream of the temperature regulator 107. It is conceivable however that the pump 110 is located upstream of the temperature regulator 107.

The pump 110 may be in communication with the sterilizing module 103 via the temperature regulator 107. The pump 110 may be configured to receive a gaseous medium which is cooled in the temperature regulator 107 so that the pressurized gas expelled after the pump 110 at the outlet 106 has a lower temperature than the heating medium. The pressurized gas may be drawn from the sterilizing module 103. A pressurized gas which is sterile may thus be provided at the outlet 106 in a facilitated manner. The pressurized gas may for example be air obtained from above a bath of sterilizing agent. Providing the pressurized gas from the sterilizing module 103 thus allows for efficiently obtaining sterile pressurized gas while the temperature regulator 107, in conjunction with the control unit 108, allows for the sterile gas to be cooled and utilized for removal of sterilizing agent residues as pressurized gas without being detrimental to the sterilization of the web 101 as explained above.

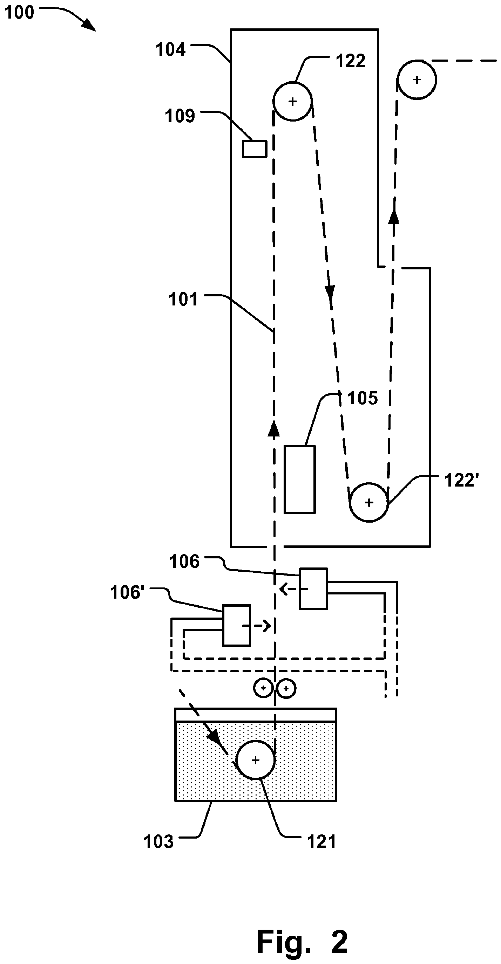

The pressurized gas outlet 106 may comprise first and second outlets 106, 106', arranged on opposite sides of the web 101 as schematically illustrated in Fig. 2. This may provide for a more efficient temperature control of the web 101, and thereby improve control of the evaporation process of the sterilization agent, and also for a more efficient removal of residues of sterilization agent on e.g. opening elements formed on the web 101.

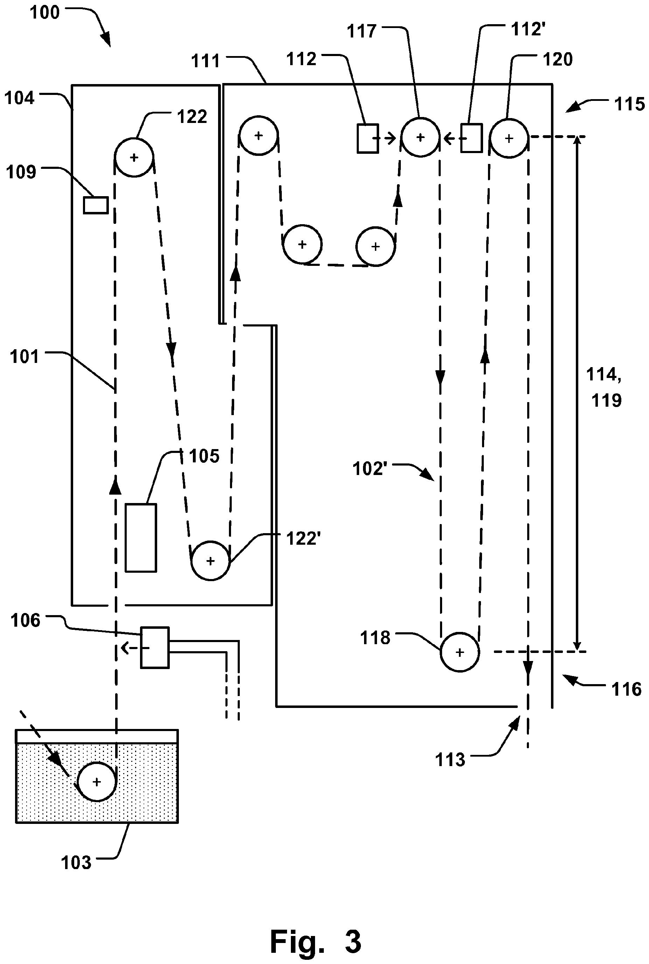

The sterilization apparatus 100 may comprise an aseptic chamber 111 arranged downstream of the sterilization chamber 104 along the path 102, 102', in which the web 101 is conveyed. The aseptic chamber 111 may comprise a unit 112, 112', configured to eject a gaseous medium towards the web 101 for removal of sterilizing agent residues, e.g. by pushing the residues off from web 101 with the pressure from the gaseous medium, or by distributing the residues for easier evaporation. A further improved removal of residues may thus be provided. Also, having a second step of exposing the web 101 to a gaseous medium for further removal of residues, allows for decreasing the temperatures in the sterilization chamber 104, thereby avoiding too fast evaporation of sterilizing agent (i.e. too short contact time between sterilization agent and the web 101) while the risk of residues of sterilizing agent at the end of the procedure is reduced. The gaseous medium may be air or any other suitable medium such as an inert gas. The unit 112, 112', may also heat the web 101 which may facilitate the evaporation of small droplets remaining on the web 101. The unit configured to eject a gaseous medium towards the web 101 for removal of sterilizing agent residues may comprise a first 112 and a second 112' arranged on opposite sides of the web 101 as schematically illustrated in Fig. 3. The process may thus be further optimized, and it is conceivable that any plurality of such units may be provided in the aseptic chamber 111.

The aseptic chamber 111 may comprise a downstream opening 113 for the web 101 to exit the aseptic chamber 111. The aseptic chamber 111 may have a longitudinal length 114, between opposite first and second ends 115, 116, along which the web 101 is conveyed in a substantially straight configuration before exiting the aseptic chamber 111 at the downstream opening 113, as schematically illustrated in Fig. 3. I.e. the longitudinal length 114 may correspond substantially to a maximum length along which the web 101 may extend in the aseptic chamber 111 without needing to change direction, e.g. turn around a conveyor element 117, 118 (such as a roller 117, 118). The aseptic chamber 111 may comprise first and second conveyor elements 117, 118, arranged adjacent each of the opposite first and second ends 115, 116. An extended path 102' along which the web 101 is conveyed pass the first and second conveyor elements 117, 118. The extended path 102' may have a length corresponding to at least twice a distance 119 between the first and second conveyor elements 117, 118. This provides for optimizing the distance the web 101 may be conveyed in the aseptic chamber 111, so that any residues of sterilizing agent have time to evaporate.

Fig. 3 illustrates an example where the path 102' has been extended even further. I.e. the sterilization apparatus 100 may comprise a third conveyor element 120 at which the extended path 102' passes. The first and third conveyor elements 117, 120, may be arranged adjacent the first end 115, and the second conveyor element 118 may be arranged adjacent the downstream opening 113 at the second end 116. The path 102' may thus have a length corresponding to at least three times the distance 119 between the second conveyor element 128 and the first or third conveyor elements 117, 120, and the web 101 may thus remain in the sterilization apparatus 100 for a further extended time, to facilitate the removal of any residues of sterilizing agent.

The unit 112, 112', configured to eject a gaseous medium towards the web 101 may be arranged adjacent the first end 115, and the first or second conveyor element 117, 118, may be arranged at the opposite second end 116 downstream of said unit 112, 112'. In the example of Fig. 3 the second conveyor element 118 is arranged opposite said unit 112, 112'.

Further, the unit 112, 112', configured to eject a gaseous medium towards the web 101 may be arranged adjacent the first end 115 and the first conveyor element 117.

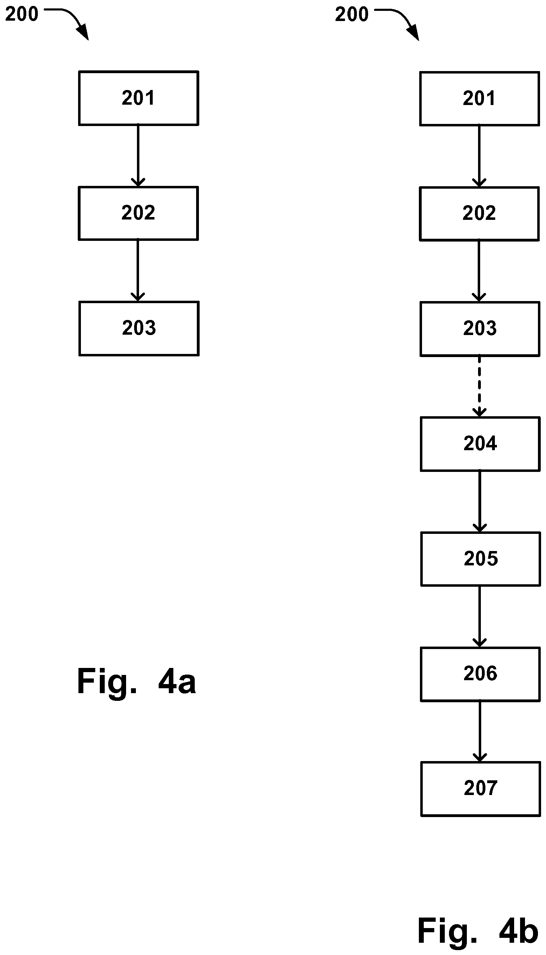

Fig. 4a illustrates a flow chart of a method 200 of sterilizing a web 101 of packaging material. The order in which the steps of the method 200 are described and illustrated should not be construed as limiting and it is conceivable that the steps can be performed in varying order. A method 200 of sterilizing a web 101 of packaging material is thus provided. The method 200 comprises applying 201 a sterilizing agent to the web 101, and expelling 202 a pressurized gas towards the web 101 subsequent of applying the sterilizing agent. The method 200 further comprises heating 203 the web 101 in a sterilizing chamber 104 with a heating medium subsequent of expelling the pressurized gas towards the web 101, wherein the pressurized gas has a lower temperature than said heating medium. The method 200 thus provides for the advantageous benefits as described above in relation to the apparatus 100 and Figs 1 - 3, thereby allowing for ensuring a controlled evaporation of the sterilizing agent and improved sterilization results of the web 101.

Fig. 6c illustrates a further flow chart of a method 200 of
The order in which the steps of the method 200 are described and illustrated should not be construed as limiting and it is conceivable that the steps can be performed in varying order. The method 200 may comprise controlling 204 the temperature of the pressurized gas based on temperature data received from the sterilization chamber 104 adjacent a path 102 in which the web 101 is conveyed for providing 205 a temperature of the web 101 of packaging material to assume predefined values along said path 102, for providing the improved sterilization process as described above.

The method 200 may comprise ejecting 206 a gaseous medium towards the web 101 for removal of sterilizing agent residues downstream of the sterilization chamber 104 along the path 102, 102' in which the web is conveyed. This provides for further removal of any residues as elucidated above.

The method 200 may comprise extending 207 the path 102' along which the web 101 is conveyed to at least twice a distance 119 between first and second conveyor elements 117, 118, arranged adjacent opposite first and second ends 115, 116, respectively, of a longitudinal length 114 of the aseptic chamber 104 along which the web 101 is conveyed in a substantially straight configuration before exiting the aseptic chamber. The time by which the web remains in the sterilizing apparatus can thus be extended for removal of sterilizing agent residues.

A packaging machine (not shown) for producing sealed packages of pourable food products from a web 101 of packaging material is also provided. The packaging machine carries out the method 200 of sterilizing a web 101 of packaging material as described above in relation to Figs. 4a-b. Alternatively, or in addition, the packaging machine comprises a sterilization apparatus 100 as described above in relation to Figs. 1 - 3. The packaging machine thus provides for the advantageous benefits as described above in relation to the apparatus 100 and the method 200, and Figs 1 - 4.

A computer program product is provided comprising instructions which, when the program is executed by a computer, cause the computer to carry out the steps of the method 200 as described above in relation to Figs. 4a-b. The computer program thus provides for the advantageous benefits as described above in relation to the method 200, and Figs 1 - 4.

The present invention has been described above with reference to specific examples. However, other examples than the above described are equally possible within the scope of the invention. The different features and steps of the invention may be combined in other combinations than those described. The scope of the invention is only limited by the appended patent claims.

More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used.

## Claims

1. A sterilization apparatus (100) for sterilizing a web (101) of packaging material being conveyed along a path (102) thereof in an upstream- to a downstream direction, the sterilization apparatus comprises:
a sterilizing module (103) in which a sterilizing agent is applied to the web,
a sterilization chamber (104) through which the web is fed when conveyed downstream of the sterilizing module,
a heating unit (105) configured to heat the web in a heating medium when fed through the sterilization chamber, and
a pressurized gas outlet (106) arranged upstream of the heating unit and downstream of the sterilizing module, wherein the pressurized gas outlet is configured to expel a pressurized gas having a lower temperature than said heating medium, towards the web.

2. Sterilization apparatus according to claim 1, comprising
a temperature regulator (107) being in communication with the pressurized gas outlet,
a control unit (108) connected to the temperature regulator and to at least one temperature sensor (109) in the sterilization chamber adjacent the path in which the web is conveyed to receive temperature data therefrom, wherein the control unit is configured to control the temperature of said pressurized gas based on the temperature data so that the temperature of the web of packaging material assume predefined values along said path in which the web is conveyed.

3. Sterilization apparatus according to claim 2, comprising
a pump (110) in communication with the pressurized gas outlet and being configured to pressurize said pressurized gas for propagation towards the web, and wherein
the control unit is configured to control the pressure of the pressurized gas being expelled from the pressurized gas outlet based on the temperature data.

4. Sterilization apparatus according to any of claims 1 - 3, wherein the pump is in communication with the sterilizing module via the temperature regulator, whereby the pump is configured to receive gas which is cooled in the temperature regulator so that pressurized gas expelled after the pump at the pressurized gas outlet has a lower temperature than said heating medium.

5. Sterilization apparatus according to any of claims 1 - 4, wherein the pressurized gas outlet comprises first and second outlets (106, 106') arranged on opposite sides of the web.

6. Sterilization apparatus according to any of claims 1 - 5, comprising
an aseptic chamber (111) arranged downstream of the sterilization chamber along the path in which the web is conveyed, wherein the aseptic chamber comprises
a unit (112, 112') configured to eject a gaseous medium towards the web for removal of sterilizing agent residues.

7. Sterilization apparatus according to claim 6, wherein the aseptic chamber comprises
a downstream opening (113) for the web to exit the aseptic chamber, wherein the aseptic chamber has a longitudinal length (114), between opposite first and second ends (115, 116), along which the web is conveyed in a substantially straight configuration before exiting the aseptic chamber,
first and second conveyor elements (117, 118) arranged adjacent each of the opposite first and second ends, an extended path (102') along which the web is conveyed passing the first and second conveyor elements, wherein
the extended path has a length corresponding to at least twice a distance (119) between the first and second conveyor elements.

8. Sterilization apparatus according to claim 7, comprising
a third conveyor element (120) at which said extended path passes, wherein the first and third conveyor elements are arranged adjacent the first end and the second conveyor element is arranged adjacent the downstream opening at the second end, whereby the path has a length corresponding to at least three times the distance (119) between the second conveyor element and the first or third conveyor elements.

9. Sterilization apparatus according to claim 7, wherein the unit configured to eject a gaseous medium towards the web is arranged adjacent the first end, and the first or second conveyor element is arranged at the opposite second end downstream of said unit.

10. Sterilization apparatus according to claim 8, wherein the unit configured to eject a gaseous medium towards the web is arranged adjacent the first end and the first conveyor element.

11. A method (200) of sterilizing a web (101) of packaging material, comprising
applying (201) a sterilizing agent to the web,
expelling (202) a pressurized gas towards the web subsequent of applying the sterilizing agent, and
heating (203) the web in a sterilizing chamber (104) with a heating medium subsequent of expelling the pressurized gas towards the web, wherein the pressurized gas has a lower temperature than said heating medium.

12. Method according to claim 11, comprising
controlling (204) the temperature of the pressurized gas based on temperature data received from the sterilization chamber adjacent a path (102) in which the web is conveyed for providing (205) a temperature of the web of packaging material to assume predefined values along said path.

13. Method according to claim 11 or 12, comprising
ejecting (206) a gaseous medium towards the web for removal of sterilizing agent residues downstream of the sterilization chamber along the path in which the web is conveyed.

14. Method according to any of claims 11 - 13, comprising
extending (207) the path (102') along which the web is conveyed to at least twice a distance (119) between first and second conveyor elements (117, 118) arranged adjacent opposite first and second ends (115, 116), respectively, of a longitudinal length (114) of the aseptic chamber along which the web is conveyed in a substantially straight configuration before exiting the aseptic chamber.

15. A packaging machine for producing sealed packages of pourable food products from a web (101) of packaging material, the packaging machine carrying out a method (200) of sterilizing a web (101) of packaging material according to any of claims 11 - 14, and/or comprises a sterilization apparatus (100) according to any of claims 1 - 10.

16. A computer program product comprising instructions which, when the program is executed by a computer, cause the computer to carry out the steps of the method (200) according to any of claims 11 - 14.
